# EUROPÄISCHE PATENTSCHRIFT

(11) **EP 0 876 382 B1**
(45) Veröffentlichungstag und Bekanntmachung des Hinweises auf die Patenterteilung: **14.03.2001**
(21) Anmeldenummer: 96944016.3
(22) Anmeldetag: 19.12.1996
(51) Int. Cl.: C07F 15/04, C07F 15/00, C08F 10/00, C08F 4/70, C08G 67/02, C07C 2/26

(54) **BIS- UND TRIS(PYRAZOLYL)BORATMETALLKOMPLEX-KATALYSATOREN**
BIS- AND TRIS(PYRAZOLYL)BORATE METAL COMPLEX CATALYSTS
COMPLEXES METALLIQUES DE BIS- ET TRIS(PYRAZOLYL)BORATE CONSTITUANT DES CATALYSEURS

(30) Priorität: 21.12.1995 DE 19548146
(43) Veröffentlichungstag der Anmeldung: 11.11.1998
(73) Patentinhaber: BASF AKTIENGESELLSCHAFT, 67056 Ludwigshafen (DE)
(72) Erfinder: KLÄUI, Wolfgang, D-40225 Düsseldorf (DE); DOMHÖVER, Bernd, D-45883 Gelsenkirchen (DE)
(86) Internationale Anmeldenummer: EP9605715
(87) Internationale Veröffentlichungsnummer: WO9723492

(56) Entgegenhaltungen:
- BE-A- 759 729
- US-A- 4 870 042
- JOURNAL OF ORGANOMETALLIC CHEMISTRY, Bd. 205, 1981, Seiten 273-279, XP000673254 MEALLI C.:
- INORGANICA CHIMICA ACTA, Bd. 224, 1994, Seiten 131-135, XP000671620 ONISHI M: "Pyrazolyl carbon..."

## Beschreibung

Die vorliegende Erfindung betrifft Metallkomplexe der allgemeinen Formeln (I) und (I'), die für die Oligomerisation und Polymerisation von olefinisch ungesättigten Verbindungen, sowie für deren Copolymerisation mit Kohlenmonoxid geeignet sind 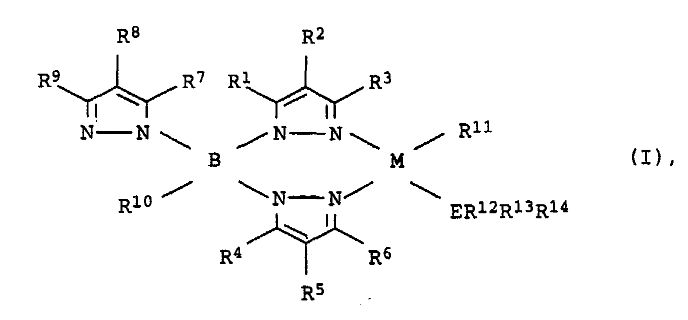 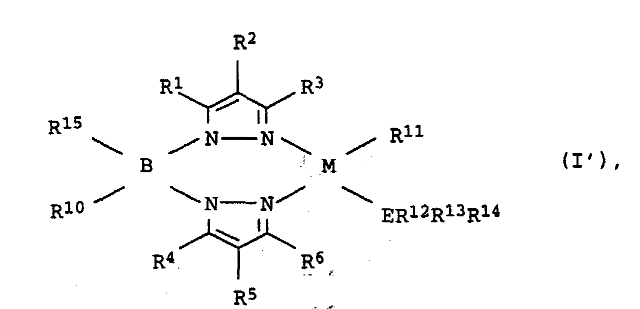 in welchen die Substituenten und Indizes die folgende Bedeutung haben:
- M: ein Metall aus der achten Nebengruppe des Periodensystems der Elemente,
- E: ein Element aus der fünften Hauptgruppe des Periodensystems der Elemente,
- R¹ bis R¹¹, R¹⁵: Substituenten ausgewählt aus der Gruppe bestehend aus Wasserstoff, C₁- bis C₃₀-kohlenstofforganischen Resten und C₃- bis C₃₀-siliziumorganischen Resten,
- R¹² bis R¹⁴: Substituenten ausgewählt aus der Gruppe bestehend aus C₁- bis C₃₀-kohlenstofforganischen Resten und C₃- bis C₃₀-siliziumorganischen Resten,
und Katalysatorsysteme, die für die Oligomerisation und Polymerisation von olefinisch ungesättigten Verbindungen, sowie deren Copolymerisation mit Kohlenmonoxid geeignet sind, enthaltend als aktive Bestandteile
A) einen Metallkomplexe der allgemeinen Formel (I) 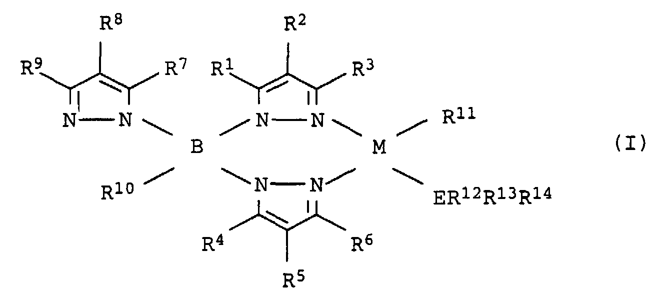 oder einen Metallkomplex der allgemeinen Formel (I') 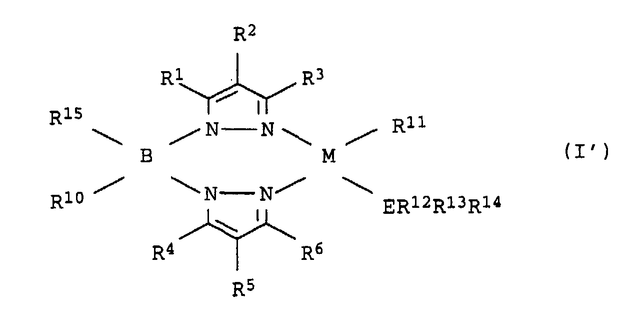 in welchen die Substituenten und Indizes die folgende Bedeutung haben:
   - M: ein Metall aus der achten Nebengruppe des Periodensystems der Elemente,
   - E: ein Element aus der fünften Hauptgruppe des Periodensystems der Elemente,
   - R¹ bis R¹¹, R¹⁵: Substituenten ausgewählt aus der Gruppe bestehend aus Wasserstoff, C₁- bis C₃₀-kohlenstofforganischen Resten und C₃- bis C₃₀-siliziumorganischen Resten,
   - R¹² bis R¹⁴: Substituenten ausgewählt aus der Gruppe bestehend aus C₁- bis C₃₀-kohlenstofforganischen Resten und C₃- bis C₃₀-siliziumorganischen Resten
   und
B) eine Lewis-Säure.

Außerdem betrifft die vorliegende Erfindung ein Verfahren zur Herstellung von Metallkomplexen der allgemeinen Formel (I) 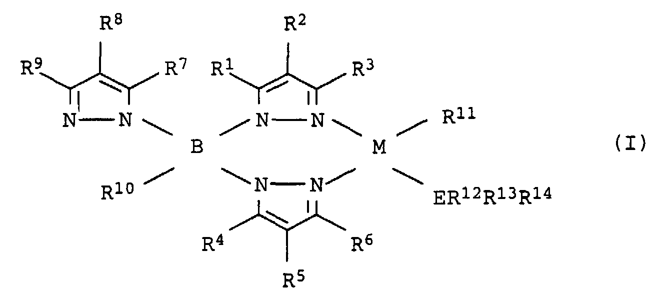 oder (I') 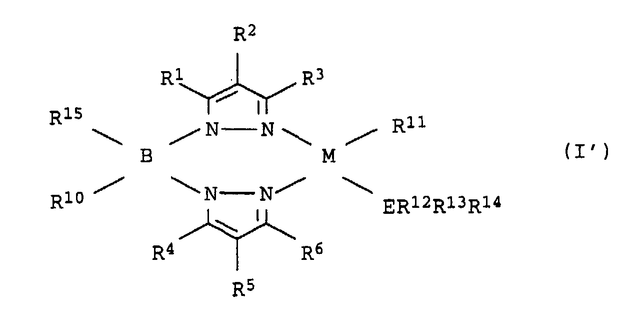 durch Umsetzung von Halogenometallkomplexen der Metalle der achten Nebengruppe des Periodensystems der Elemente mit Tris(pyrazolyl)boratanionen der allgemeinen Formel (II) 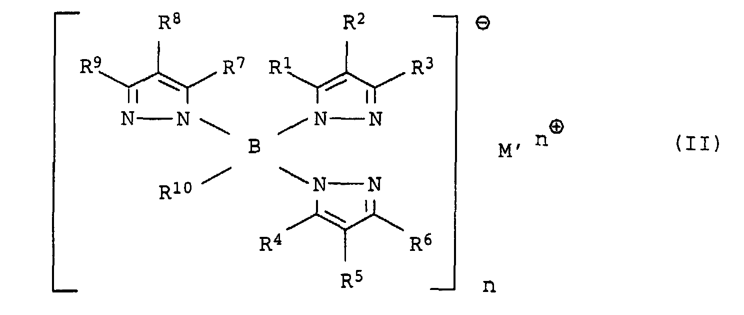 oder mit Bis(pyrazolyl)boratanionen der allgemeinen Formel (II') 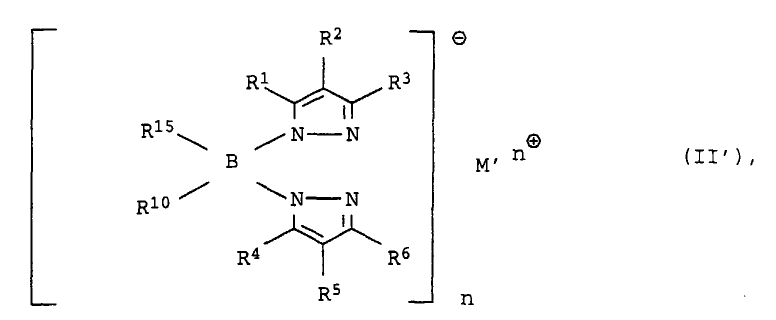 wobei die Substituenten und Indizes in (I), (I'), (II) oder (II') die folgenden Bedeutung haben:
- M: ein Metall aus der achten Nebengruppe des Periodensystems der Elemente,
- M': Lithium, Natrium, Kalium, Rubidium, Cäsium, Magnesium, Calcium, Thallium,
- E: ein Element aus der fünften Hauptgruppe des Periodensystems der Elemente,
- R¹ bis R¹¹: Substituenten ausgewählt aus der Gruppe bestehend aus Wasserstoff, C₁- bis C₃₀-kohlenstofforganischen Resten und C₃- bis C₃₀-siliziumorganischen Resten,
- R¹² bis R¹⁴, R¹⁵: Substituenten ausgewählt aus der Gruppe bestehend aus C₁- bis C₃₀-kohlenstofforganischen Resten und C₃- bis C₃₀₉-siliziumorganischen Resten,
- n: 1 oder 2, wobei n die formale Wertigkeit von M' ist,
ein Verfahren zur Herstellung von Oligomerisaten und Polymerisaten von olefinisch ungesättigten Verbindungen und von Copolymerisaten aus olefinisch ungesättigten Verbindungen und Kohlenmonoxid durch Polymerisation der Monomeren bei einer Temperatur im Bereich von 0 bis 300°C und einem Druck im Bereich von 1 bis 500 000 kPa in Gegenwart eines Metallkomplexes der allgemeinen Formeln (I) 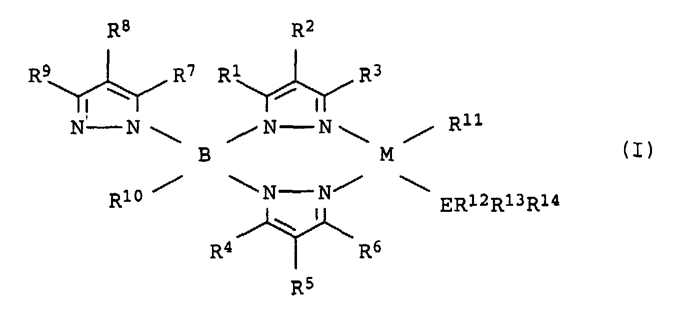 oder (I') 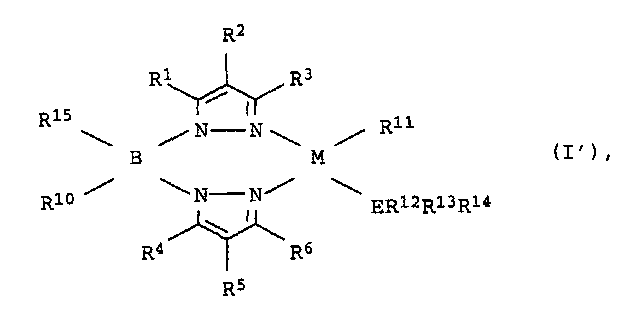 in welchen die Substituenten und Indizes die folgende Bedeutung haben:
- M: ein Metall aus der achten Nebengruppe des Periodensystems der Elemente,
- E: ein Element aus der fünften Hauptgruppe des Periodensystems der Elemente,
- R¹ bis R¹¹, R¹⁵: Substituenten ausgewählt aus der Gruppe bestehend aus Wasserstoff, C₁- bis C₃₀-kohlenstofforganischen Resten und C₃- bis C₃₀-siliziumorganischen Resten,
- R¹² bis R¹⁴: Substituenten ausgewählt aus der Gruppe bestehend aus C₁- bis C₃₀-kohlenstofforganischen Resten und C₃- bis C₃₀-siliziumorganischen Resten
und ein Verfahren zur Herstellung von Oligomerisaten und Polymerisaten von olefinisch ungesättigten Verbindungen und von Copolymerisaten aus olefinisch ungesättigten Verbindungen und Kohlenmonoxid durch Polymerisation der Monomeren bei einer Temperatur im Bereich von 0 bis 300°C und einem Druck im Bereich von 1 bis 500 000 kPa in Gegenwart eines Katalysatorsystems, enthaltend als aktive Bestandteile
A) einen Metallkomplex der allgemeinen Formel (I) 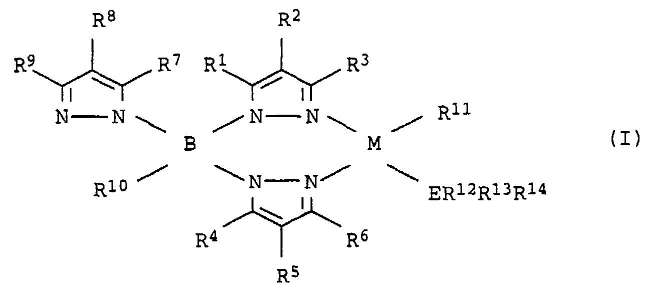 oder (I') 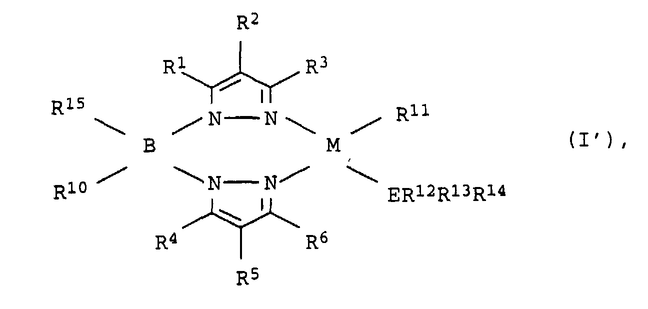 in welcher die Substituenten und Indizes die folgende Bedeutung haben:
   - M: ein Metall aus der achten Nebengruppe des Periodensystems der Elemente,
   - E: ein Element aus der fünften Hauptgruppe des Periodensystems der Elemente,
   - R¹ bis R¹¹, R¹⁵: Substituenten ausgewählt aus der Gruppe bestehend aus Wasserstoff, C₁- bis C₃₀-kohlenstofforganischen Resten und C₃- bis C₃₀-siliziumorganischen Resten
   - R¹² bis R¹⁴: Substituenten ausgewählt aus der Gruppe bestehend aus C₁- bis C₃₀-kohlenstofforganischen Resten und C₃- bis C₃₀-siliziumorganischen Resten
   und
B) eine Lewis-Säure.

Weiterhin betrifft die vorliegende Erfindung die Verwendung der Metallkomplexe (I) und (I') gemäß Anspruch 1 als Katalysatoren zur Herstellung von Oligomerisaten und Polymerisaten aus olefinisch ungesättigten Verbindungen und zur Herstellung von Copolymerisaten aus olefinisch ungesättigten Verbindungen und Kohlenmonoxid sowie die Verwendung der Katalysatorsysteme gemäß Anspruch 4 zur Herstellung von Oligomerisaten und Polymerisaten aus olefinisch ungesättigten Verbindungen und zur Herstellung von Copolymerisaten aus olefinisch ungesättigten Verbindungen und Kohlenmonoxid.

Metallkomplexe der Metalle der achten Nebengruppe des Periodensystems der Elemente sind bisher, wie Nickel, bei Olefinoligomerisationen (W. Keim, Angew. Chem. (1990), Seite 251 ff) oder auch bei Olefin-Kohlenmonoxid-Copolymerisationen, Palladium, (E. Drent et al., in "Ziegler-Natta Catalysts", G. Fink, R. Mühlhaupt, H.H. Brintzinger (Herausgeber), Springer Verlag Berlin (1995), Seite 482 ff) oder Nickel (US 3 984 388; US 5 214 126), verwendet worden. BE 759729 betrifft Pyrazolylboratmetallkomplexe zur Hydroformylierung von Olefinen.

Keiner der eingesetzten Metallkomplexe oder Katalysatoren war jedoch frei von Nachteilen; sei es, daß er aufwendig herzustellen war, zu teuer war, unbefriedigende Aktivitäten besaß oder daß er zu hohe Polymerisationstemperaturen oder Polymerisationsdrücke erforderte.

Aufgabe der vorliegenden Erfindung war es daher Metallkomplexe (I) und (I') und Katalysatorsysteme enthaltend (I) oder (I') zur Verfügung zu stellen, die die genannten Nachteile nicht, oder nur in untergeordnetem Maße aufweisen, eine hohe Produktivität haben und leicht zugänglich sind. Der vorliegenden Erfindung lag ferner die Aufgabe zugrunde ein Verfahren zur Herstellung der Metallkomplexe (I) und (I') zur Verfügung zu stellen, sowie ein Verfahren zur Herstellung von Oligomerisaten und Polymerisaten aus olefinisch ungesättigten Verbindungen und ein Verfahren zur Herstellung von Copolymerisaten aus olefinisch ungesättigten Verbindungen und Kohlenmonoxid in Gegenwart der Metallkomplexe (I) oder (I') oder der Katalysatorsysteme und die Verwendung der Metallkomplexe (I) oder (I') oder der Katalysatorsysteme zur Herstellung derartiger Oligomerisate und Polymerisate zur Verfügung zu stellen.

Demgemäß wurden die eingangs definierten Metallkomplexe (I) und (I') und die eingangs definierten Katalysatorsysteme gefunden.

Außerdem wurde ein Verfahren zur Herstellung der Metallkomplexe (I) und (I'), ein Verfahren zur Herstellung von Oligomerisaten und Polymerisaten von olefinisch ungesättigten Verbindungen und von Copolymerisaten aus olefinisch ungesättigten Verbindungen und Kohlenmonoxid in Gegenwart der Metallkomplexe (I) oder (I') oder der eingangs definierten Katalysatorsysteme gefunden.

Weiterhin wurde die Verwendung der Metallkomplexe (I) und (I') und die Verwendung der eingangs definierten Katalysatorsysteme zur Herstellung von Oligomerisaten und Polymerisaten aus olefinisch ungesättigten Verbindungen oder zur Herstellung von Copolymerisaten aus eben diesen Verbindungen und Kohlenmonoxid gefunden.

Als Metalle M der Metallkomplexe der allgemeinen Formeln (I) und (I') eignen sich die Metalle der achten Nebengruppe (VIIIB) des Periodensystems der Elemente, also Eisen, Cobalt, Ruthenium, Rhodium, Osmium, Iridium, Platin, Palladium sowie ganz besonders Nickel. Die Metalle können in den Komplexen formal zweifach positiv geladen vorliegen.

Als Elemente E kommen die Elemente der V. Hauptgruppe des Periodensystems der Elemente (Gruppe VA), also Stickstoff, Phosphor, Arsen, Antimon oder Bismut in Betracht. Besonders geeignet sind Stickstoff oder Phosphor, insbesondere Phosphor.

Die Reste R¹ bis R¹¹ und R¹⁵ können Wasserstoff oder kohlenstoff- oder siliziumorganische Reste sein. Als kohlenstofforganische Reste R¹ bis R¹¹ und R¹⁵ kommen aliphatische, sowie cycloaliphatische und ganz besonders aromatische, mit jeweils 1 bis 30 Kohlenstoffatomen in Betracht.

Exemplarisch seien genannt lineare und vorzugsweise verzweigte C₁-bis C₁₀-Alkylgruppen, wie Methyl, Ethyl, 1-Propyl, iso-Propyl, 1-Butyl, sec.-Butyl, tert.-Butyl und 1-Hexyl. Als geeignete cycloaliphatsiche Reste seien die C₃- bis C₆-Cycloalkylreste wie Cyclohexyl genannt.

Besonders gut eignen sich als kohlenstofforganische Reste R¹ bis R¹¹ und R¹⁵ aromatische Reste, die auch substituiert sein können, beispielsweise mit C₁- bis C₆-Alkylresten, mit weiteren C₆-C₁₀-Arylresten oder mit Halogenen wie Fluor, Chlor, Brom, Iod oder aber mit Perfluoralkylresten wie Trifluormethyl-, Pentafluorethyl-, Heptafluorpropyl- oder Heptafluorisopropylresten. Tragen die Arylreste keine weiteren Kohlenstoffsubstituenten, so haben sie vorzugsweise eine Kohlenstoffanzahl von 6 bis 20. Mit Alkylgruppen oder deren Derivaten substituierte Alkylarylreste R¹ bis R¹¹ haben vorzugsweise 1 bis 10 Kohlenstoffatome im Alkylrest und 6 bis 10 Kohlenstoffatome im Arylrest.

Der aromatische Ring kann selbstverständlich auch mehrfach substituiert sein.

Beispielhaft seien als gut geeignete Reste R¹ bis R¹¹ genannt Phenyl, 1-Naphthyl, 2-Naphthyl, ortho-Tolyl, meta-Tolyl, p-Tolyl, 2,4,6-Trimethylphenyl (Mesityl), 2-(Trifluormethyl)phenyl, 2,6-Di(trifluormethyl)phenyl, 2,6-Dimethylphenyl, 2,4-Dimethylphenyl.

Als siliziumorganische Reste R¹ bis R¹¹ und R¹⁵ mit 3 bis 30 Kohlenstoffatomen kommen vorzugsweise Triorganosilylreste mit C₁-bis C₁₀-Alkylresten oder C₆-C₁₀-Arylresten in Frage, wie Trimethylsilyl, Triethylsilyl, Triphenylsilyl oder tert.-Butyldimethylsilyl.

Die Reste R¹², R¹³ und R¹⁴ können die gleiche Bedeutung wie die kohlenstofforganischen und siliziumorganischen Reste R¹ bis R¹¹ haben. Die Reste R¹², R¹³ oder R¹⁴ sind in der Regel jedoch nicht Wasserstoff.

Als sehr gut geeignete Metallkomplexe (I) und (I') haben sich solche erwiesen, worin R¹¹, R¹², R¹³, R¹⁴, R³ und R⁶ und gegebenenfalls auch R⁹ aromatische Reste der bereits für diese Substituenten spezifizierten Typen sind, vorzugsweise Phenyl oder ortho-Tolyl, R¹⁰, R¹⁵ und die Substituenten an den Pyrazolylringen R¹, R², R⁴, R⁵, R⁷ und R⁸ Wasserstoff bedeuten.

Als Beispiele für ganz besonders bevorzugte Metallkomplexe (I) seien [Hydrotris(3-phenylpyrazolyl)borat) (ortho-Tolyl) (triphenylphosphan)]nickel-(II), [Hydrotris (3-p-tolylpyrazolyl)borat) (ortho-Tolyl)(triphenylphosphan)]nickel-(II), Hydrotris(3-phenylpyrazolyl)borat) (ortho-Tolyl) (tri(p-tolyl)phosphan)]nickel- (II) genannt.

Als besonders bevorzugter Metallkomplex (I') ist insbesondere zu nennen [{Dihydrobis(3-phenylpyrazolyl)borat} (ortho-tolyl) (triphenylphosphin)nickel-(II)].

Es hat sich als vorteilhaft herausgestellt, die erfindungsgemäßen Metallkomplexe (I) und (I') mit einer Verbindung B) umzusetzen, welche in der Lage ist, den Liganden ER¹²R¹³R¹⁴ stärker an sich zu binden, als dies das Metall M vermag.

Hierfür geeignete Verbindungen sind in der Regel Lewis-Säuren, welche nach Römpp Chemie Lexikon, Georg Thieme Verlag Stuttgart, New York (1990), Seite 2499, Elektronenpaarakzeptoren darstellen.

Die chemische Natur der Lewis-Säuren B) ist in der Regel nicht kritisch. Gut geeignet sind die Halogenide des Magnesiums, Bors, Aluminiums, Galliums, Siliziums, Germaniums, Zinns, Phosphors, Arsens, Antimons, Titans, Zirkoniums und Hafniums, wobei die Metalle vorzugsweise in ihrer höchsten formalen Oxidationsstufe vorliegen.

Weiterhin sind geeignete Lewis-Säuren Carbokationensalze, wie Triphenylmethyltetrafluoroborat.

Beispielhaft seien als besonders gut geeignete Lewis-Säuren, Bortrifluorid, Bortrifluorid-Diethylether-Komplex, Bortribromid, Tris(pentafluorphenyl)bor, Aluminiumtrichlorid und Magnesiumdichlorid genannt.

Gute Ergebnisse, insbesondere bei der Oligomerisation und Polymerisation von olefinisch ungesättigten Verbindungen, wurden mit Bortrifluorid-Diethylether-Komplex als Komponente B) erzielt.

Das molare Verhältnis von Komponente A) zur Lewis-Säure B) beträgt im allgemeinen 0,01:1 bis 1:1, vorzugsweise 0,02:1 bis 1:1.

Die Katalysatorsysteme können als Komponente A) selbstverständlich auch Gemische unterschiedlicher Metallkomplexe (I) oder (I') enthalten.

Die Herstellung der erfindungsgemäßen Metallkomplexe (I) und (I') erfolgt vorteilhaft durch Substitution eines Halogenatoms, also Fluor, Chlor, Brom oder Iod, in einem Halogenmetallkomplex der Metalle M, gegen einen Bis- bzw. Tris(pyrazolyl)borat-Liganden.

Hierzu setzt man üblicherweise, vorzugsweise in einem organischen Lösungsmittel wie Dichlormethan, Toluol, Tetrahydrofuran oder Diethylether, eine Hauptgruppenmetallverbindung (II) 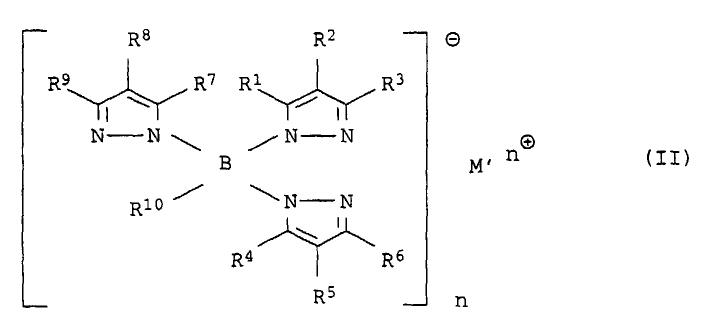 oder (II') 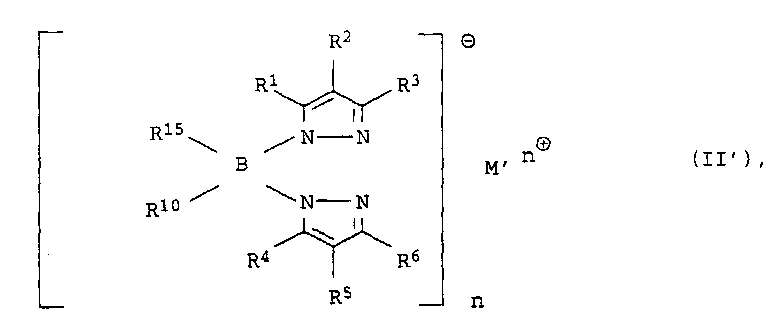 in welchen M' Lithium, Natrium, Kalium, Rubidium, Cäsium, Magnesium, Calcium und vorzugsweise Thallium bedeuten, n als formale Wertigkeit von M' 1 oder 2 bedeutet und die Reste R¹ bis R¹⁵ die bereits für (I) bzw. (I') spezifizierte Bedeutung haben, mit einem Halogenmetallkomplex der Metalle M, insbesondere Nickel oder Palladium, um.

Für die Herstellung der Metallkomplexe (I') werden bevorzugt auch Lösungsmittelgemische, beispielsweise Aceton/Dichlormethan, verwendet.

Vorzugsweise verwendet man als Halogenmetallkomplex einen solchen der allgemeinen Formel M(ER¹²R¹³R¹⁴)₂(R¹¹)X(III), wobei M, E, R¹¹ bis R¹⁴ die eingangs für (I) bzw. (I') definierte Bedeutung haben und X Fluor, Chlor, Brom, Iod, vorzugsweise Chlor oder Brom bedeutet.

Die Umsetzung wird im allgemeinen bei einer Temperatur im Bereich von (-)80°C bis 200°C, vorzugsweise im Bereich von 0°C bis 110°C durchgeführt.

Die erfindungsgemäßen Metallkomplexe (I) oder (I') oder die Katalysatorsysteme können zur Herstellung von Oligomeren und Polymeren aus olefinisch ungesättigten Verbindungen sowie zur Herstellung von, im allgemeinen alternierenden, Copolymeren aus olefinisch ungesättigten Monomeren und Kohlenmonoxid (Polyketone) verwendet werden.

Als olefinisch ungesättigte Verbindungen kommen grundsätzlich alle Monomere dieser Verbindungsklasse in Betracht.

Bevorzugt sind Ethylen und C₃- bis C₁₀-Alk-1-ene wie 1-Buten, 1-Hexen und hauptsächlich Propen, außerdem Butadien, sowie daneben Cycloolefine wie Cyclopenten, Cyclohexen, Norbornen und Norbornadien und seine Derivate.

Unter den olefinisch ungesättigten aromatischen Monomeren seien in erster Linie Styrol und α-Methylstyrol genannt.

Bedeutung haben ferner Acrylsäure und Methacrylsäure sowie deren Derivate, darunter insbesondere die Nitrile, die Amide und die C₁-C₆-Alkylester, wie beispielsweise Ethylacrylat, n-Butylacrylat, tert.-Butylacrylat, Methylmethacrylat.

Weitere geeignete Monomere sind Vinylchlorid, Vinylacetat, Vinylpropionat, Maleinsäureanhydrid und N-Vinylpyrrolidon.

Selbstverständlich können auch Mischungen verschiedener Monomerer eingesetzt werden, wobei das Mischungsverhältnis im allgemeinen nicht kritisch ist.

Das molare Verhältnis der olefinisch ungesättigten Verbindungen, vorzugsweise Ethylen, Propen, 1-Buten oder 1-Hexen zu Kohlenmonoxid kann weitgehend frei gewählt werden und beträgt vorzugsweise zwischen 0,01 : 1 bis 100 : 1, bevorzugt in der Nähe von 1:1.

Die Polymerisationen zur Herstellung der Oligomeren und Polymeren aus olefinisch ungesättigten Verbindungen, sowie der Kohlenmonoxid-Copolymeren können sowohl absatzweise als auch kontinuierlich durchgeführt werden.

Drücke von 1 bis 500000 kPa, vorzugsweise 200 bis 350000 kPa und insbesondere 500 bis 30000 kPa, Temperaturen von 0 bis 300°C, bevorzugt 20 bis 250°C und insbesondere 40 bis 150 °C haben sich als geeignet erwiesen.

Polymerisationsreaktionen mit Hilfe der eingangs definierten Metallkomplexe (I) oder (I') oder Katalysatorsysteme lassen sich in der Gasphase, in Suspension, in flüssigen und in überkritischen Monomeren und in, unter den Polymerisationsbedingungen inerten, Lösungsmitteln durchführen.

Geeignete inerte Lösungsmittel sind Alkohole wie Methanol, Ethanol, Propanol, i-Propanol, 1-Butanol und tert.-Butanol, Sulfoxide und Sulfone, beispielsweise Dimethylsulfoxid, Ester wie Essigester und Butyrolacton, Ether wie Tetrahydrofuran, Dimethylethylenglycol und Diisopropylether sowie vorzugsweise aromatische Lösungsmittel wie Benzol, Toluol, Ethylbenzol oder Chlorbenzol oder Gemische derselben.

Das Molekulargewicht der erfindungsgemäßen Polymere läßt sich durch die Variation der Polymerisationstemperatur, durch protische Verbindungen wie Alkohole, beispielsweise Methanol, Ethanol, tert.-Butanol, vorzugsweise Methanol und durch den Zusatz von Wasserstoff in dem Fachmann bekannter Weise beeinflussen. Im allgemeinen bewirkt eine hohe Konzentration regelnder Substanzen und/oder eine hohe Polymerisationstemperatur ein relativ geringes Molekulargewicht und vice versa.

Die Oligomeren, vorzugsweise solche der C₂- bis C₁₀-Alk-1-ene, wie Ethylen, Propen, haben im allgemeinen einen Polymerisationsgrad, bestimmt über Endgruppenanalyse im ¹³C-NMR-Spektrum, von 2 bis 1000, vorzugsweise 2 bis 100 und insbesondere 2 bis 50.

Die Hochpolymeren, vorzugsweise die der C₂- bis C₁₀-Alk-1-ene wie Ethen, Propen, 1-Buten, 1-Hexen, 1-Octen haben im allgemeinen ein Molekulargewicht Mw, bestimmt mit der Methode der Gelpermeationschromatographie bei 135°C in 1,2,4-Trichlorbenzol gegen Polyethylenstandard im Bereich von 30000 bis 3000000 und ein Mw/Mn im allgemeinen im Bereich von 1,5 bis 5,0.

Die Molekulargewichte Mw (Gewichtsmittelwert) der Kohlenmonoxid-Copolymeren (gemessen mit der Methode der Gelpermeationschromatographie (GPC) bei 25°C mit Shodex® HFIP 803 bzw. 805 als Säulenmaterial und Hexafluorisopropanol als Lösungsmittel gegen Polymethylmethacrylat-Standard) liegen im allgemeinen im Bereich von 1000 bis 1000000, bevorzugt 1000 bis 100000.

Die Molekulargewichtsverteilung Mw/Mn (Gewichtsmittelwert/Zahlenmittelwert), gemessen mit der Methode der Gelpermeationschromatographie (GPC) analog vorangegangener Beschreibung, der Kohlenmonoxid-Copolymere beträgt im allgemeinen 1 bis 50, vorzugsweise 1 bis 20.

Die erfindungsgemäßen Kohlenmonoxid-Copolymerisate lassen sich aufgrund ihrer zahlreichen funktionellen Gruppen mit den üblichen chemischen Reaktionen, wie zum Beispiel EP-A 372 602 beschrieben, oder einer Kombination beider Methoden modifizieren.

### Beispiele

### Beispiel 1: Synthese von [{Hydrotris(3-phenylpyrazolyl)borat} (ortho-tolyl) (triphenylphosphan)nickel(II)]

Bei Raumtemperatur wurden unter Stickstoff-Atmosphäre 2.00 g (2.7 mmol) [NiBr(o-tol)(PPh₃)₂] in 25 ml Dichlormethan gelöst und mit 1.71 g (2.7 mmol) [Tl{HB(3-Ph-pyrazolyl]₃}] versetzt. Man rührte die Lösung 60 min und filtrierte das entstandene TlBr ab. Nach der Zugabe von 10 ml Hexan engte man die Lösung im Vakuum bis auf ca. 5 ml ein und wusch den entstandenen, gelben Feststoff solange mit wenig Hexan, bis das Lösungsmittel farblos blieb. Anschließend wurde der Komplex in wenig Toluol gelöst, filtriert, und nach der Zugabe von 10 ml Hexan bis zur Trockene eingeengt. Das analysenreine Produkt kann aus Diethylether umkristallisiert werden.
Ausbeute: 1.89 g (82 %).
¹H-NMR (CDCl₃): 1.60 ppm (s, 3 H, CH₃-Tolyl), 5.51 ppm (dt, 1 H, Tolyl), 5.78 ppm (d, 1 H, pz, ³J^{HH} ∼ 2.3 Hz), 6.04 ppm (d, 1 H, pz, ³J_{HH} ∼ 2.1 Hz), 6.20 ppm (m, 2 H, Tolyl), 6.52 ppm (d, 2 H, J ∼ 8.1 Hz), 6.69 ppm (m, 6 H), 6.87 ppm (d, 1 H, pz, ³J_{HH} ∼ 2.1 Hz), 7.06 ppm (m, 6 H), 7.41 ppm (m, 19 H), 8.01 ppm (d, 2 H, J ∼ 7.2 Hz), 8.28 ppm (d, 1 H, pz, ³J_{HH} ∼ 2.2 Hz) (d₈-Toluol) : 2.13 ppm (s, 3 H, CH₃-Tolyl), 5,83 ppm (d, 1 H, pz, ³J_{HH} ∼ 2.1 Hz) , 6.51 ppm (m, 2 H, Tolyl), 6.94 - 7.61 ppm (m, H), 7.81 ppm, (m, H), 8.36 ppm (d, 2 H, J ∼ 7.1 Hz), 8.42 ppm (d, 1 H, pz, ³J_{HH} ∼ 2.2 Hz)
¹³C{¹H}-NMR: 23.2 ppm, 103.1 ppm, 106.3 ppm, 107.1 ppm, 119.8 ppm, 122.7 ppm, 126.7 ppm, 127.7 ppm (d, J_{PC} ∼ 13.9 Hz), 128.0 ppm, 128.2 ppm (d, J_{PC} ∼ 9.7 Hz), 128.5 ppm, 129.1 ppm, 129.3 ppm, 130.0 ppm, 130.1 ppm, 130.4 ppm, 130.7 ppm, 134.5 ppm (d, J_{PC} ∼ 9.8 Hz), 135.0 ppm, 135.1 ppm, 135.4 ppm, 135.7 ppm, 139.6 ppm, 140.1 ppm (d, J_{PC} ∼ 5.2 Hz), 146.3 ppm.
³¹P{¹H}-NMR: 18.4 ppm (s)
IR (KBr): 2382 ν(BH) w, 1468 w, 1438 w, 1433 w, 1371 w, 1280 w, 1207 m, 1180 w, 1103 w, 1088 w, 1070 w, 1051 w, 1038 w, 751 s, 736 w, 696 s, 530 m.

| Elementaranalyse: (C₅₂H₄₄N₆BPNi) (C4H₁₀O), | | | |
|---|---|---|---|
| berechnet | C 72,51 | H 5,87 | N 9,06; |
| gefunden | C 72,12 | H 5,81 | N 9,07. |

### Beispiele 2 bis 4: Katalytische Oligomerisierungen

102 mg (0,12 mmol) [Hydrotris(3-phenylpyrazolyl)borat) (ortho-tolyl) (triphenylphosphan)nickel-II] wurde in 50 ml Toluol gelöst, gegebenenfalls wurden hierzu 0,6 ml (4,1 mmol, B:Ni = 35:1) Bortrifluorid-Diethyletherkomplex zugegeben, und dann wurde unter einem Monomerdruck von 4000 kPa 16 Stunden lang oligomerisiert.

Die Reaktionsprodukte wurden mittels Gaschromatographie (GC/MS-Kopplung) analysiert (Säule: Ultra 2 crosslinked, beschichtet mit 5 % Phenylsilikon, 95 % Methylsilikon; 25 m x 0,2 mm; 50 bis 200°C; 5°C/min; Stickstoffträgergas).

Versuchsparameter und Ergebnisse sind Tabelle 1 zu entnehmen.

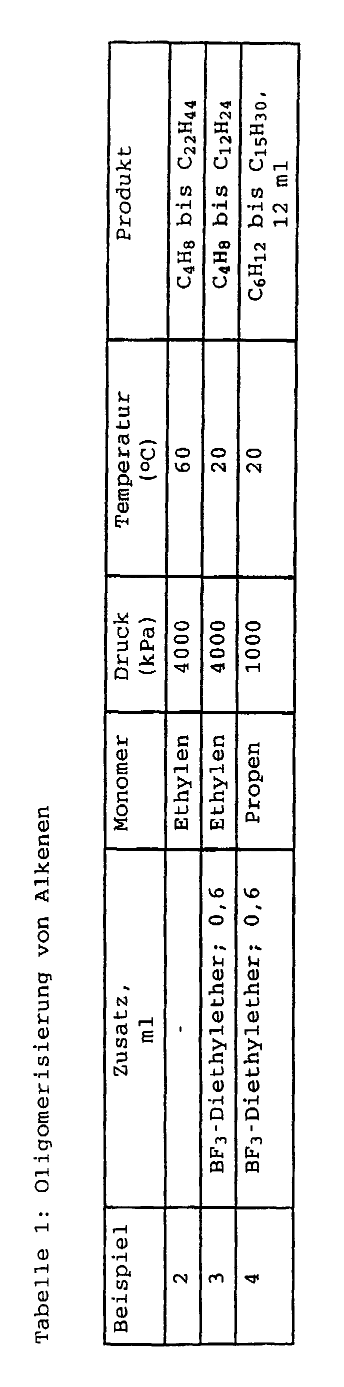

### Beispiel 5: Polyketonherstellung

102 mg (0,12 mmol) [Hydrotris(3-phenylpyrazolyl)borat) (ortho-tolyl)(triphenylphosphin)nickel-II] wurden in 50 ml Toluol bei 60°C in Anwesenheit von 0,3 mol Ethylen (4000 kPa) und 14,3 mmol Kohlenmonoxid (350 kPa) 16 Stunden lang umgesetzt. Danach wurde durch Entfernen des Lösungsmittels 1,15 g Polyketon isoliert (IR: ν_{c=o} = 1691, ¹³C-NMR: 36,4 ppm, Singulett; 212,7 ppm, Singulett. M_{w} = 5770, M_{w}/Mₙ = 1.3).

### Beispiel 6

### Synthese von [{Dihydrobis-3-phenylpyrazolyl)borat} (ortho-tolyl)(triphenylphosphin)nickel-(II)]

Äquimolare Mengen von K[H₂B(3-Ph-pyrazolyl)₂] (223.9 mg, 0.66 mmol) und [NiBr(o-tol)(PPh₃)₂] (500.0 mg, 0.66 mmol) wurden unter Stickstoffatmosphäre 16 Stunden in einem Gemisch aus Aceton/Dichlormethan (15 ml/15 ml) gerührt. Der entstandene Niederschlag wurde abfiltiert und das Filtrat nach Zugabe von ca. 5 ml n-Hexan im Vakuum bis auf 5 ml eingeengt, wobei ein gelber Niederschlag ausfiel, der mit wenig Hexan ausgewaschen wurde, bis das Lösungsmittel hellgelb bis farblos blieb. Der Niederschlag wurde im Vakuum getrocknet und anschließend mehrere Male mit wenig Aceton gewaschen, um paramagnetische Verunreinigungen zu entfernen. Das Produkt wurde im Vakuum getrocknet.
Ausbeute: 130 mg (28 %)
¹H-NMR (CDCl₃): 2.20 ppm (s, 3H, CH₃, o-tol), 5.70 ppm (d, 1H, pz, ³J_{HH ∼} 2,3 Hz), 6.00 ppm (d, 2H, o-tol, ³J_{HH ∼} 6.4 Hz),
6.25-6.37 ppm (m, 3H, o-tol), 7.01 ppm (d, 1H, pz, ³J_{HH ∼} 2.2 Hz), 7.1-8.3 ppm (d+m+d, 27H, PPh₃, pz, Ph);
³¹P{¹H}-NMR (CDCl₃): 22.56 ppm (s, PPh₃);
IR (KBr): 2420 cm⁻¹ νBH)

### Beispiel 7

### Polyketonherstellung

56 mg (0.10 mmol) [{Dihydrobis-3-phenylpyrazolyl)borat} (ortho-tolyl) (triphenylphosphin)nickel-(II)] wurden in 10 ml Toluol gelöst und in einem Autoklaven (100 ml) unter einem Ethendruck von 40 bar und einem Kohlenmonoxid-Druck von 3.5 bar 16 Stunden bei 60°C gerührt. Das ausgefallene Polyketon wurde abfiltriert und mit Tetrahydrofuran gewaschen.
Ausbeute: 1.05 g

## Patentansprüche

1. Metallkomplexe der allgemeinen Formeln (I) oder (I'), die für die Oligomerisation und Polymerisation von olefinisch ungesättigten Verbindungen, sowie für deren Copolymerisation mit Kohlenmonoxid geeignet sind 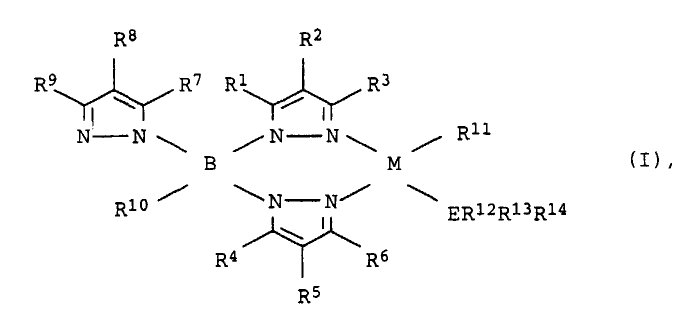 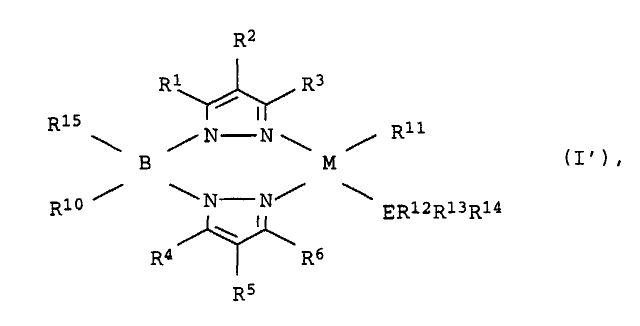 in welchen die Substituenten und Indizes die folgende Bedeutung haben:
M ein Metall aus der achten Nebengruppe des Periodensystems der Elemente,
E ein Element aus der fünften Hauptgruppe des Periodensystems der Elemente,
R¹ bis R¹¹, R¹⁵ Substituenten ausgewählt aus der Gruppe bestehend aus Wasserstoff, C₁- bis C₃₀-kohlenstofforganischen Resten und C₃- bis C₃₀-siliziumorganischen Resten,
R¹² bis R¹⁴ Substituenten ausgewählt aus der Gruppe bestehend aus C₁- bis C₃₀-kohlenstofforganischen Resten und C₃- bis C₃₀-siliziumorganischen Resten.

2. Metallkomplexe nach Anspruch 1, wobei M Nickel bedeutet.

3. Metallkomplexe nach den Ansprüchen 1 oder 2, wobei R³, R⁶, R⁹ und R¹¹ bis R¹⁴ C₆- bis C₂₀-Aryl oder C₇- bis C₃₀-Alkylaryl, mit 1 bis 10 Kohlenstoffatomen im Alkylrest und 6 bis 10 Kohlenstoffatomen im Arylrest und R¹⁰ und R¹⁵ Wasserstoff bedeuten.

4. Katalysatorsysteme, die für die Oligomerisation und Polymerisation von olefinisch ungesättigten Verbindungen, sowie deren Copolymerisation mit Kohlenmonoxid geeignet sind, enthaltend als aktive Bestandteile
A) einen Metallkomplexe der allgemeinen Formel (I) 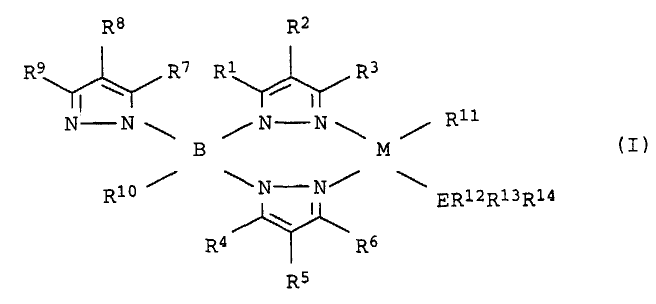 oder einen Metallkomplex der allgemeinen Formel (I') 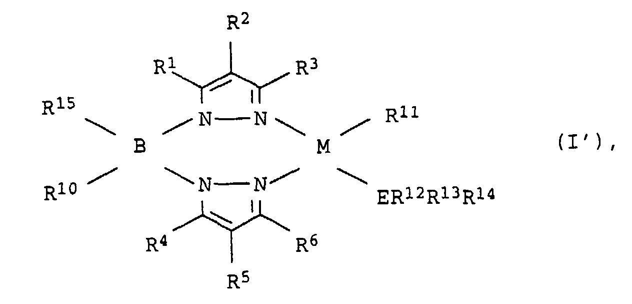 in welchen die Substituenten und Indizes die folgende Bedeutung haben:
M ein Metall aus der achten Nebengruppe des Periodensystems der Elemente,
E ein Element aus der fünften Hauptgruppe des Periodensystems der Elemente,
R¹ bis R¹¹, R¹⁵ Substituenten ausgewählt aus der Gruppe bestehend aus Wasserstoff, C₁- bis C₃₀-kohlenstofforganischen Resten und C₃-bis C₃₀-siliziumorganischen Resten,
R¹² bis R¹⁴ Substituenten ausgewählt aus der Gruppe bestehend aus C₁- bis C₃₀-kohlenstofforganischen Resten und C₃- bis C₃₀-siliziumorganischen Resten
und
B) eine Lewis-Säure.

5. Verfahren zur Herstellung von Metallkomplexen der allgemeinen Formeln (I) 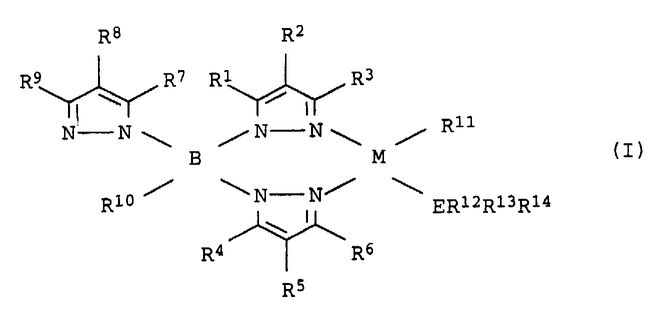 oder (I') 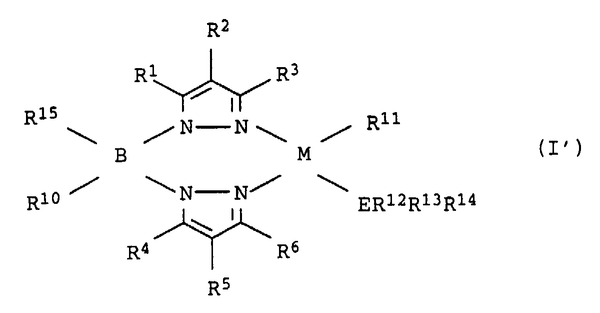 durch Umsetzung von Halogenometallkomplexen der Metalle der achten Nebengruppe des Periodensystems der Elemente mit Tris(pyrazolyl)boratanionen der allgemeinen Formel (II) 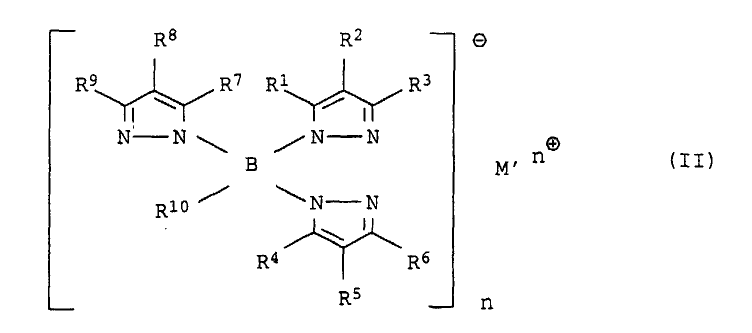 oder mit Bis(pyrazolyl)boratanionen der allgemeinen Fromel (II') 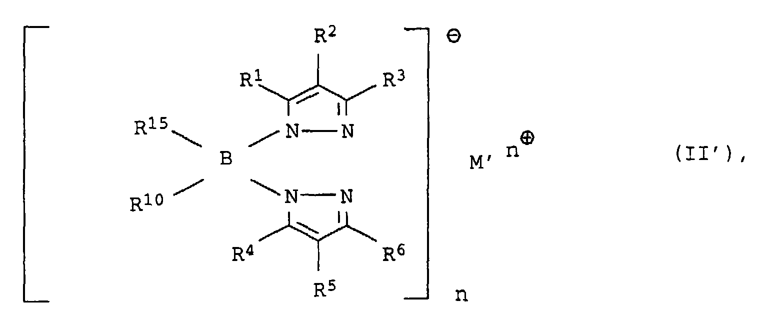 wobei die Substituenten und Indizes in (I), (I'), (II) oder (II') die folgenden Bedeutung haben:
M ein Metall aus der achten Nebengruppe des Periodensystems der Elemente,
M' Lithium, Natrium, Kalium, Rubidium, Cäsium, Magnesium, Calcium, Thallium,
E ein Element aus der fünften Hauptgruppe des Periodensystems der Elemente,
R¹ bis R¹¹, R¹⁵ Substituenten ausgewählt aus der Gruppe bestehend aus Wasserstoff, C₁- bis C₃₀-kohlenstofforganischen Resten und C₃- bis C₃₀-siliziumorganischen Resten,
R¹² bis R¹⁴ Substituenten ausgewählt aus der Gruppe bestehend aus C₁- bis C₃₀-kohlenstofforganischen Resten und C₃- bis C₃₀₉-siliziumorganischen Resten,
n 1 oder 2, wobei n die formale Wertigkeit von M' ist.

6. Verfahren zur Herstellung von Oligomerisaten und Polymerisaten von olefinisch ungesättigten Verbindungen und von Copolymerisaten aus olefinisch ungesättigten Verbindungen und Kohlenmonoxid durch Polymerisation der Monomeren bei einer Temperatur im Bereich von 0 bis 300°C und einem Druck im Bereich von 1 bis 500 000 kPa in Gegenwart eines Metallkomplexes der allgemeinen Formeln (I) 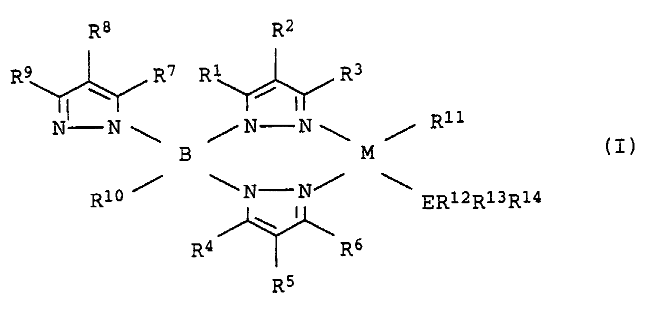 oder (I') 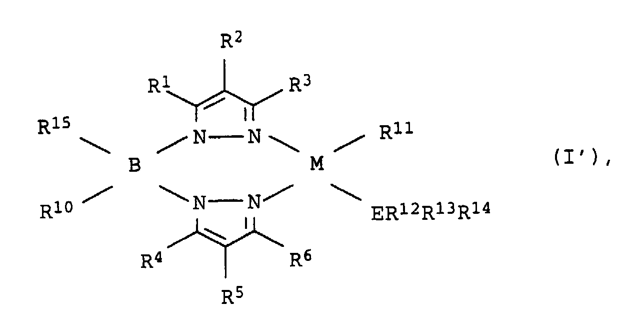 in welchen die Substituenten und Indizes die folgende Bedeutung haben:
M ein Metall aus der achten Nebengruppe des Periodensystems der Elemente,
E ein Element aus der fünften Hauptgruppe des Periodensystems der Elemente,
R¹ bis R¹¹, R¹⁵ Substituenten ausgewählt aus der Gruppe bestehend aus Wasserstoff, C₁- bis C₃₀-kohlenstofforganischen Resten und C₃- bis C₃₀-siliziumorganischen Resten,
R¹² bis R¹⁴ Substituenten ausgewählt aus der Gruppe bestehend aus C₁- bis C₃₀-kohlenstofforganischen Resten und C₃- bis C₃₀-siliziumorganischen Resten.

7. Verfahren zur Herstellung von Oligomerisaten und Polymerisaten von olefinisch ungesättigten Verbindungen und von Copolymerisaten aus olefinisch ungesättigten Verbindungen und Kohlenmonoxid durch Polymerisation der Monomeren bei einer Temperatur im Bereich von 0 bis 300°C und einem Druck im Bereich von 1 bis 500 000 kPa in Gegenwart eines Katalysatorsystems, enthaltend als aktive Bestandteile
A) einen Metallkomplexe der allgemeinen Formel (I) 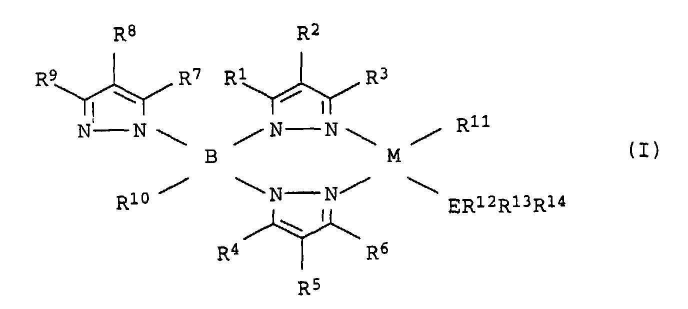 oder (I') 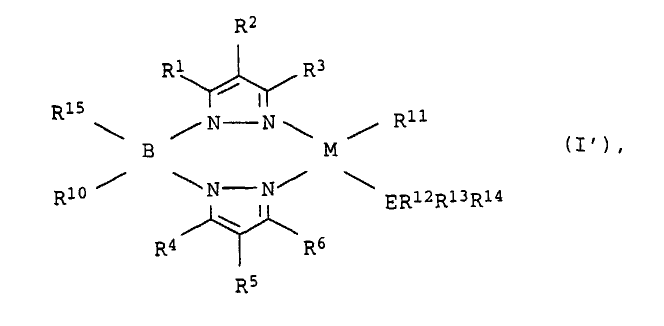 in welchen die Substituenten und Indizes die folgende Bedeutung haben:
M ein Metall aus der achten Nebengruppe des Periodensystems der Elemente,
E ein Element aus der fünften Hauptgruppe des Periodensystems der Elemente,
R¹ bis R¹¹, R¹⁵ Substituenten ausgewählt aus der Gruppe bestehend aus Wasserstoff, C₁- bis C₃₀-kohlenstofforganischen Resten und C₃-bis C₃₀-siliziumorganischen Resten
R¹² bis R¹⁴ Substituenten ausgewählt aus der Gruppe bestehend aus C₁- bis C₃₀-kohlenstofforganischen Resten und C₃- bis C₃₀-siliziumorganischen Resten
und
B) eine Lewis-Säure.

8. Verwendung der Metallkomplexe (I) oder (I') gemäß Anspruch 1 als Katalysatoren zur Herstellung von Oligomerisaten und Polymerisaten aus olefinisch ungesättigten Verbindungen und zur Herstellung von Copolymerisaten aus olefinisch ungesättigten Verbindungen und Kohlenmonoxid.

9. Verwendung der Katalysatorsysteme gemäß Anspruch 4 zur Herstellung von Oligomerisaten und Polymerisaten aus olefinisch ungesättigten Verbindungen und zur Herstellung von Copolymerisaten aus olefinisch ungesättigten Verbindungen und Kohlenmonoxid.

## Claims

1. A metal complex of the formulae (I) or (I') which is suitable for the oligomerization and polymerization of olefinically unsaturated compounds and for the copolymerization thereof with carbon monoxide 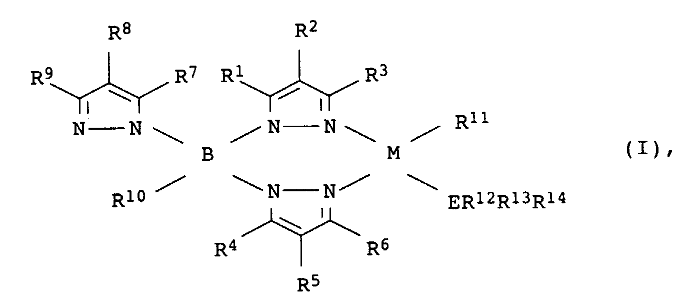 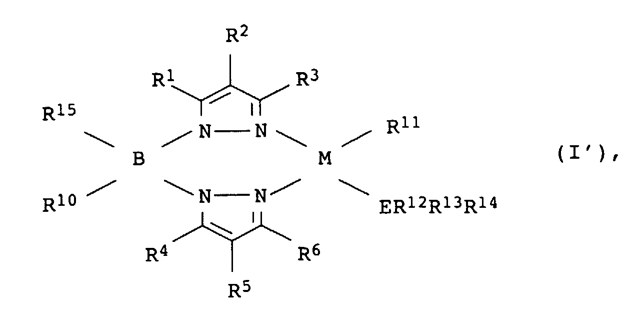 where
M is a metal from sub-group eight of the Periodic Table of the Elements,
E is an element from main group five of the Periodic Table of the Elements,
R¹ to R¹¹, R¹⁵ are substituents selected from the group consisting of hydrogen, C₁- to C₃₀-organocarbon radicals and C₃- to C₃₀-organosilicon radicals, and
R¹² to R¹⁴ are substituents selected from the group consisting of C₁- to C₃₀-organocarbon radicals and C₃- to C₃₀-organosilicon radicals.

2. A metal complex as claimed in claim 1, where M is nickel.

3. A metal complex as claimed in claim 1 or 2, where R³, R⁶, R⁹ and R¹¹ to R¹⁴ are C₆- to C₂₀-aryl or C₇- to C₃₀-alkylaryl having 1 to 10 carbon atoms in the alkyl radical and 6 to 10 carbon atoms in the aryl radical, and R¹⁰ and R¹⁵ are hydrogen.

4. A catalyst system which is suitable for the oligomerization and polymerization of olefinically unsaturated compounds and the copolymerization thereof with carbon monoxide, compri-sing, as active constituents,
A) a metal complex of the formula (I) 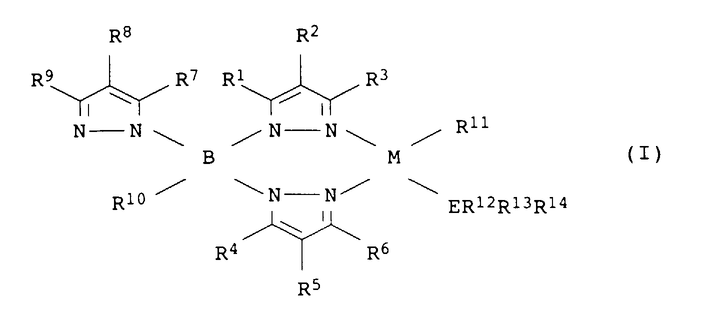 or a metal complex of the formula (I') 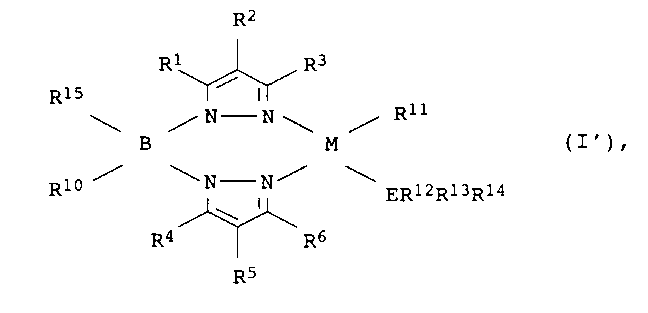 where
M is a metal from sub-group eight of the Periodic Table of the Elements,
E is an element from main group five of the Periodic Table of the Elements,
R¹ to R¹¹, R¹⁵ are substituents selected from the group consisting of hydrogen, C₁- to C₃₀-organocarbon radicals and C₃- to C₃₀-organosilicon radicals, and
R¹² to R¹⁴ are substituents selected from the group consisting of C₁- to C₃₀-organocarbon radicals and C₃- to C₃₀-organosilicon radicals,
and
B) a Lewis acid.

5. A process for the preparation of a metal complex of the formulae (I) 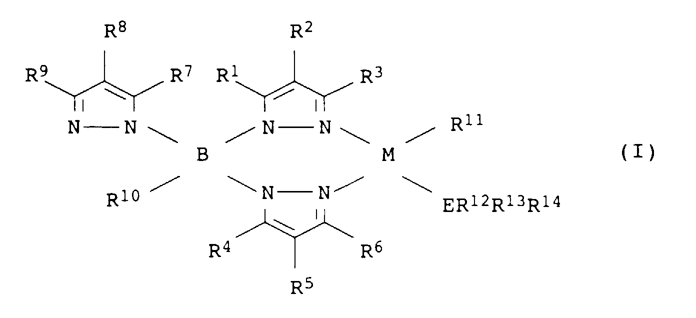 or (I') 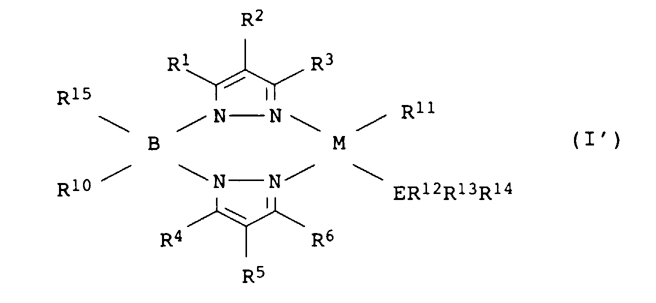 by reacting a halometal complex of the metal from sub-group eight of the Periodic Table of the Elements with a tris-(pyrazolyl)borate anion of the formula (II) 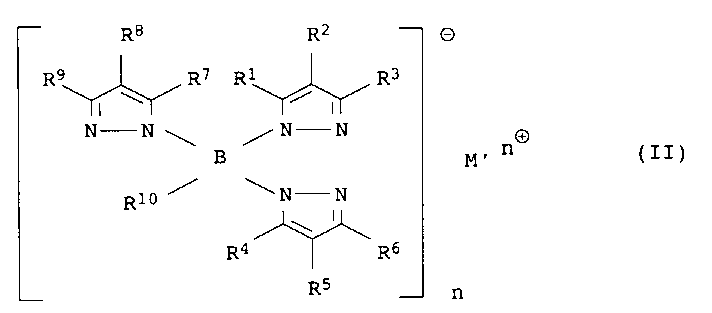 or with a bis(pyrazolyl)borate anion of the formula (II') 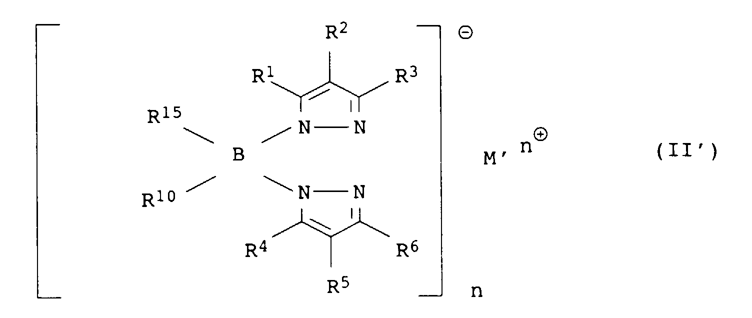 where, in (I), (I'), (II) or (II'),
M is a metal from sub-group eight of the Periodic Table of the Elements,
M' is lithium, sodium, potassium, rubidium, cesium, magnesium, calcium or thallium,
E is an element from main group five of the Periodic Table of the Elements,
R¹ to R¹¹, R¹⁵ are substituents selected from the group consisting of hydrogen, C₁- to C₃₀-organocarbon adicals and C₃- to C₃₀-organosilicon radicals,
R¹² to R¹⁴ are substituents selected from the group consisting of C₁- to C₃₀-organocarbon radicals and C₃- to C₃₀-organosilicon radicals, and
n is 1 or 2, where n is the formal valence of M'.

6. A process for the preparation of oligomers and polymers of olefinically unsaturated compounds and of copolymers of olefinically unsaturated compounds and carbon monoxide by polymerizing the monomers at from 0 to 300°C and from 1 to 500 000 kPa in the presence of a metal complex of the formulae (I) 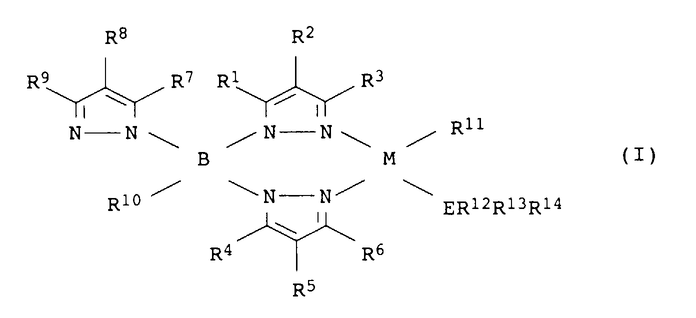 or (I') 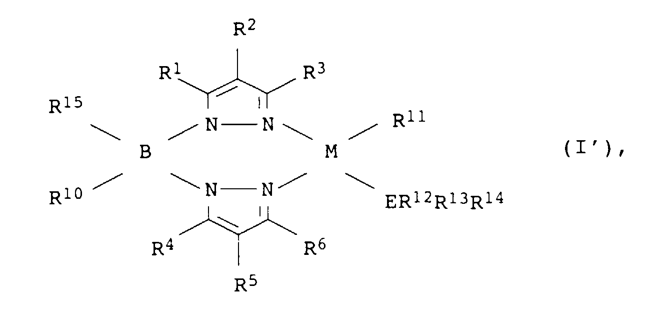 where
M is a metal from sub-group eight of the Periodic Table of the Elements,
E is an element from main group five of the Periodic Table of the Elements,
R¹ to R¹¹, R¹⁵ are substituents selected from the group consisting of hydrogen, C₁- to C₃₀-organocarbon radicals and C₃- to C₃₀-organosilicon radicals, and
R¹² to R¹⁴ are substituents selected from the group consisting of C₁- to C₃₀-organocarbon radicals and C₃- to C₃₀-organosilicon radicals.

7. A process for the preparation of oligomers and polymers of olefinically unsaturated compounds and of copolymers of olefinically unsaturated compounds and carbon monoxide by polymerizing the monomers at from 0 to 300°C and from 1 to 500 000 kPa in the presence of a catalyst system comprising, as active constituents,
A) a metal complex of the formula (I) 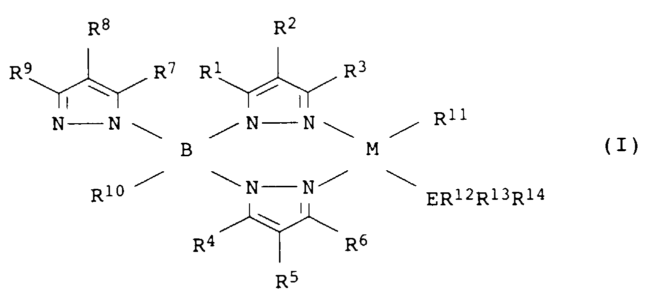 or (I') 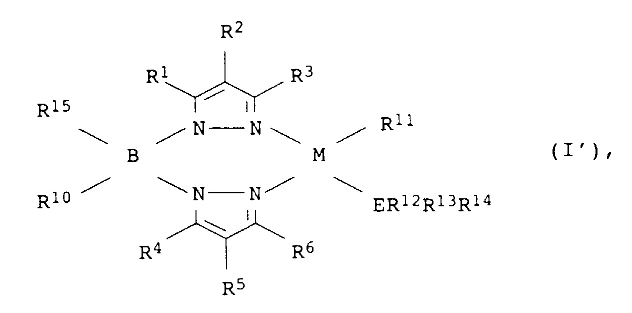 where
M is a metal from sub-group eight of the Periodic Table of the Elements,
E is an element from main group five of the Periodic Table of the Elements,
R¹ to R¹¹, R¹⁵ are substituents selected from the group consisting of hydrogen, C₁- to C₃₀-organocarbon radicals and C₃- to C₃₀-organosilicon radicals, and
R¹² to R¹⁴ are substituents selected from the group consisting of C₁- to C₃₀-organocarbon radicals and C₃- to C₃₀-organosilicon radicals,
and
B) a Lewis acid.

8. The use of a metal complex (I) or (I') as claimed in claim 1 as catalyst for the preparation of oligomers and polymers of olefinically unsaturated compounds and for the preparation of copolymers of olefinically unsaturated compounds and carbon monoxide.

9. The use of a catalyst system as claimed in claim 4 for the preparation of oligomers and polymers of olefinically unsaturated compounds and for the preparation of copolymers of olefinically unsaturated compounds and carbon monoxide.

## Revendications

1. Complexes métalliques des formules générales (I) ou (I'), convenant pour l'oligomérisation et la polymérisation de composés oléfiniquement insaturés, ainsi que pour leur copolymérisation avec du monoxyde de carbone 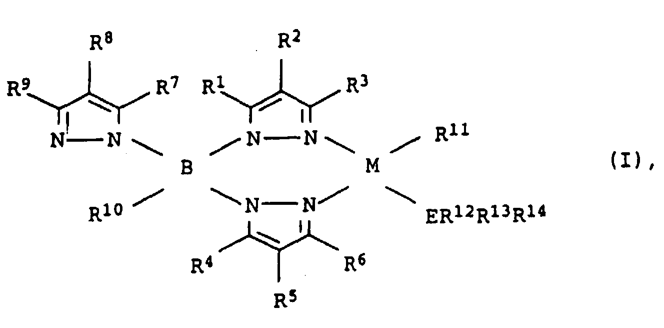 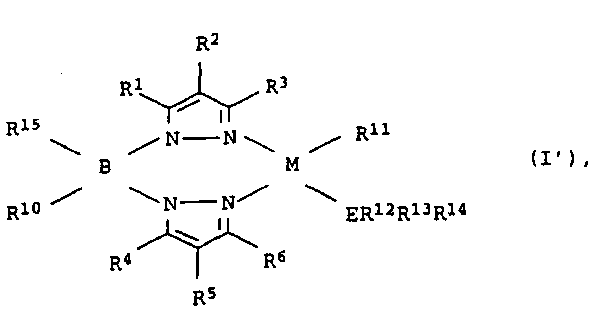 où les substituants et indices ont la signification suivante:
M représente un métal du huitième sous-groupe du système périodique des éléments,
E représente un élément du cinquième groupe principal du système périodique des éléments,
R¹ à R¹¹, R¹⁵ sont des substituants choisis dans le groupe formé par l'hydrogène, des restes organiques carbonés en C₁ à C₃₀ et des restes organosiliciques en C₃ à C₃₀,
R¹² à R¹⁴ sont des substituants choisis dans le groupe formé par des restes organiques carbonés en C₁ à C₃₀ et des restes organosiliciques en C₃ à C₃₀.

2. Complexes métalliques selon la revendication 1, où M représente le nickel.

3. Complexes métalliques selon les revendications 1 ou 2, où R³, R⁶, R⁹ et R¹¹ à R¹⁴ représentent des radicaux aryle en C₆ à C₂₀ ou alkylaryle en C₇ à C₃₀, comportant de 1 à 10 atomes de carbone dans le radical alkyle et de 6 à 10 atomes de carbone dans le radical aryle, et R¹⁰ et R¹⁵ représentent l'hydrogène.

4. Systèmes catalytiques convenant pour l'oligomérisation et la polymérisation de composés oléfiniquement insaturés, ainsi que leur copolymérisation avec du monoxyde de carbone, contenant en tant que constituants actifs:
A) un complexe métallique de la formule générale (I) 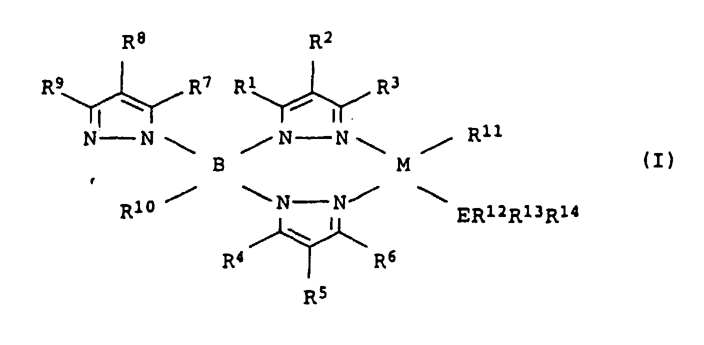 ou un complexe métallique de la formule générale (I') 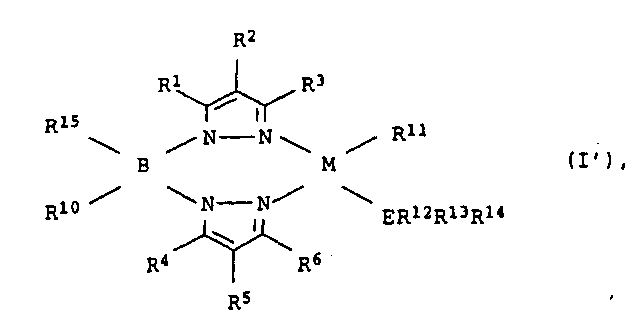 où les substituants et indices ont la signification suivante:
M représente un métal du huitième sous-groupe du système périodique des éléments,
E représente un élément du cinquième groupe principal du système périodique des éléments,
R¹ à R¹¹, R¹⁵ sont des substituants choisis dans le groupe formé par l'hydrogène, des restes organiques carbonés en C₁ à C₃₀ et des restes organosiliciques en C₃ à C₃₀,
R¹² à R¹⁴ sont des substituants choisis dans le groupe formé par des restes organiques carbonés en C₁ à C₃₀ et des restes organosiliciques en C₃ à C₃₀
et
B) un acide de Lewis.

5. Procédé de préparation de complexes métalliques des formules générales (I) 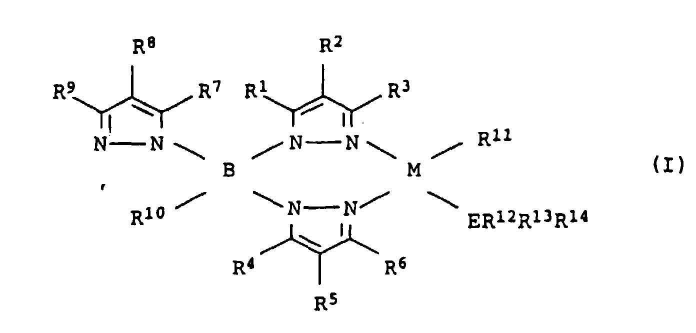 ou (I') 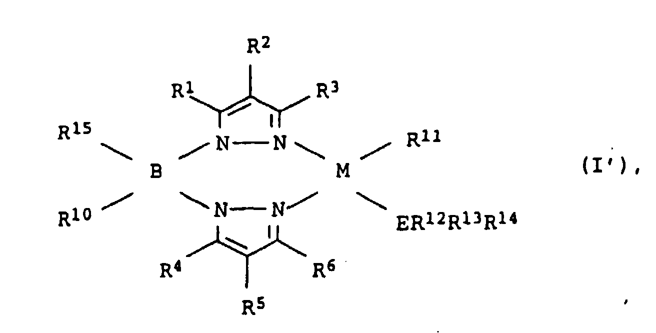 par réaction de complexes métalliques halogénés des métaux du huitième sous-groupe du système périodique des éléments avec des anions de tris(pyrazolyl)borate de la formule générale (II) 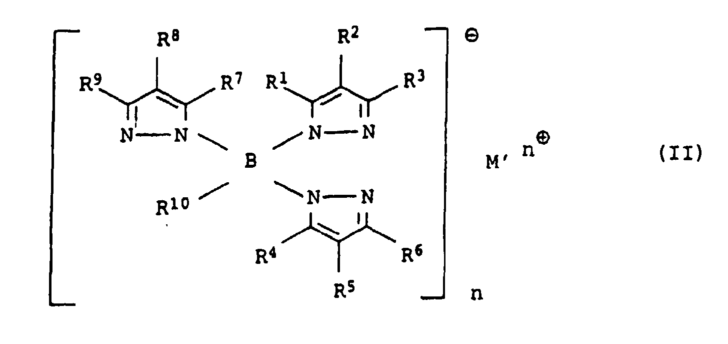 ou avec des anions de bis(pyrazolyl)borane de la formule générale (II') 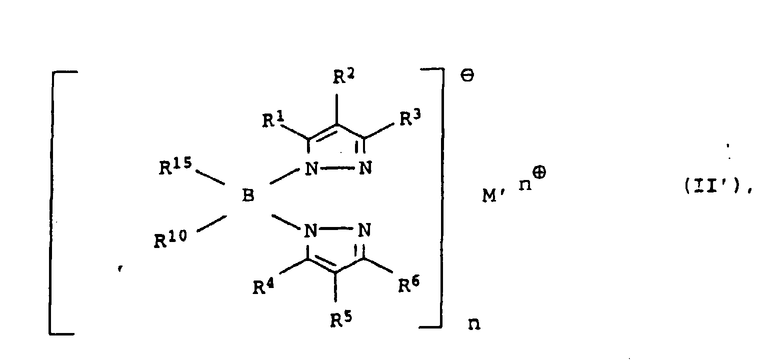 où les substituants et indices de (I), (I'), (II) ou (II') ont la signification suivante:
M représente un métal du huitième sous-groupe du système périodique des éléments,
M' représente le lithium, le sodium, le potassium, le rubidium, le césium, le magnésium, le calcium, le thallium,
E représente un élément du cinquième groupe principal du système périodique des éléments,
R¹ à R¹¹, R¹⁵ sont des substituants choisis dans le groupe formé par l'hydrogène, des restes organiques carbonés en C₁ à C₃₀ et des restes organosiliciques en C₃ à C₃₀,
R¹² à R¹⁴ sont des substituants choisis dans le groupe formé par des restes organiques carbonés en C₁ à C₃₀ et des restes organosiliciques en C₃ à C₃₀,
n a une valeur de 1 ou 2, n représentant la valence formelle de M'.

6. Procédé de préparation d'oligomères et de polymères de composés oléfiniquement insaturés, et de copolymères de composés oléfiniquement insaturés et de monoxyde de carbone, par polymérisation des monomères à une température de l'ordre de 0 à 300°C et une pression de l'ordre de 1 à 500.000 kPa, en présence d'un complexe métallique de la formule générale (I) 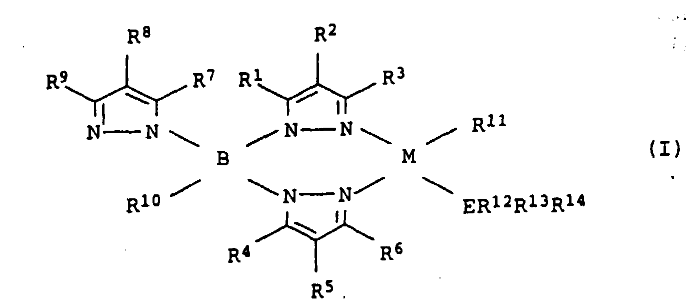 ou (I') 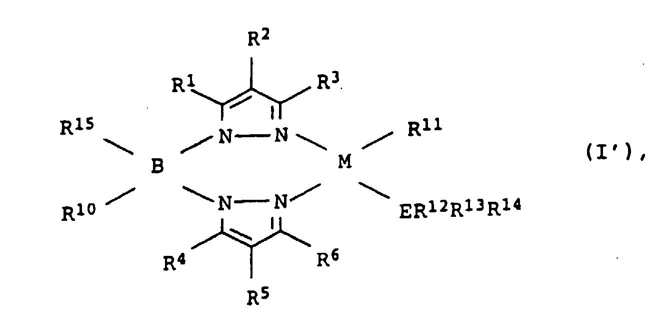 où les substituants et indices ont la signification suivante:
M représente un métal du huitième sous-groupe du système périodique des éléments,
E représente un élément du cinquième groupe principal du système périodique des éléments,
R¹ à R¹¹, R¹⁵ sont des substituants choisis dans le groupe formé par l'hydrogène, des restes organiques carbonés en C₁ à C₃₀ et des restes organosiliciques en C₃ à C₃₀,
R¹² à R¹⁴ sont des substituants choisis dans le groupe formé par des restes organiques carbonés en C₁ à C₃₀ et des restes organosiliciques en C₃ à C₃₀.

7. Procédé de préparation d'oligomères et de polymères de composés oléfiniquement insaturés, et de copolymères de composés oléfiniquement insaturés et de monoxyde de carbone, par polymérisation des monomères à une température de l'ordre de 0 à 300°C et une pression de l'ordre de 1 à 500.000 kPa, en présence d'un système catalytique contenant, en tant que constituants actifs:
A) un complexe métallique de la formule générale (I) 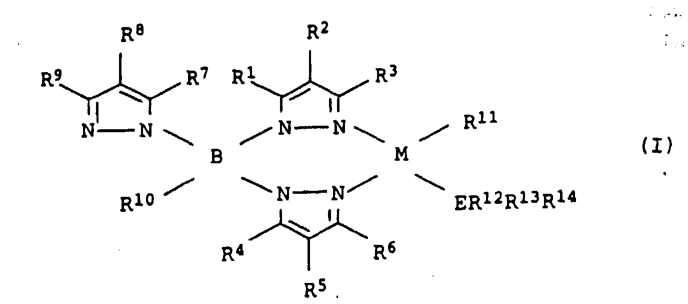 ou (I') 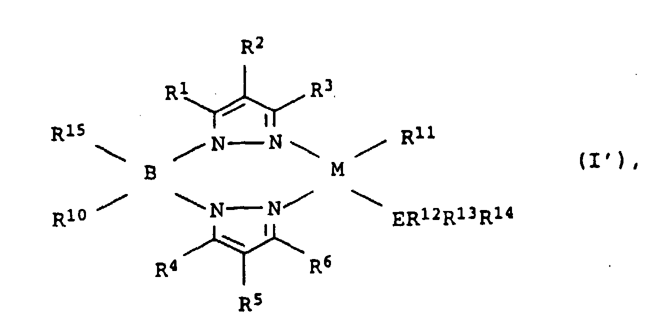 où les substituants et indices ont la signification suivante:
M représente un métal du huitième sous-groupe du système périodique des éléments,
E représente un élément du cinquième groupe principal du système périodique des éléments,
R¹ à R¹¹, R¹⁵ sont des substituants choisis dans le groupe formé par l'hydrogène, des restes organiques carbonés en C₁ à C₃₀ et des restes organosiliciques en C₃ à C₃₀,
R¹² à R¹⁴ sont des substituants choisis dans le groupe formé par des restes organiques carbonés en C₁ à C₃₀ et des restes organosiliciques en C₃ à C₃₀
et
B) un acide de Lewis.

8. Utilisation des complexes métalliques (I) ou (I') selon la revendication 1 comme catalyseurs pour la préparation d'oligomères et de polymères de composés oléfiniquement insaturés et pour la préparation de copolymères de composés oléfiniquement insaturés et de monoxyde de carbone.

9. Utilisation des systèmes catalytiques selon la revendication 4 pour la préparation d'oligomères et de polymères de composés oléfiniquement insaturés et pour la préparation de copolymères de composés oléfiniquement insaturés et de monoxyde de carbone.
